(19)

Europäisches
Patentamt
European
Patent Office
Office européen
des brevets

(11) **EP 2 323 548 B1**

(12) **EUROPEAN PATENT SPECIFICATION**

(45) Date of publication and mention
of the grant of the patent:
**14.03.2012 Bulletin 2012/11**

(21) Application number: **09749941.2**

(22) Date of filing: **25.05.2009**

(51) Int Cl.:
**A61B 5/03** (2006.01)

(86) International application number:
**PCT/EP2009/056306**

(87) International publication number:
**WO 2009/141451 (26.11.2009 Gazette 2009/48)**

(54) **NON-INVASIVE BRAIN INJURY EVALUATION**

NICHT-INVASIVE UNTERSUCHUNG VON GEHIRNVERLETZUNGEN

ÉVALUATION NON INVASIVE DE LÉSIONS CÉRÉBRALES

(84) Designated Contracting States:
**AT BE BG CH CY CZ DE DK EE ES FI FR GB GR
HR HU IE IS IT LI LT LU LV MC MK MT NL NO PL
PT RO SE SI SK TR**

(30) Priority: **23.05.2008 US 128784 P
19.05.2009 US 454538**

(43) Date of publication of application:
**25.05.2011 Bulletin 2011/21**

(73) Proprietor: **Nibie AB
11432 Stockholm (SE)**

(72) Inventors:
• **Von Holgst, Hans
162 66 Djursholm (SE)**
• **Kleiven, Svein
112 50 Stockholm (SE)**
• **Ho, Johnson
114 37 Stockholm (SE)**

(74) Representative: **Andersson, Inga-Lill
Awapatent AB
P.O. Box 45086
104 30 Stockholm (SE)**

(56) References cited:
• **HORGAN T J ET AL: "The creation of three-dimensional finite element models for simulating head impact biomechanics" INTERNATIONAL JOURNAL OF CRASHWORTHINESS, WOODHEAD PUBLISHING, CA, vol. 8, no. 4, 1 January 2003 (2003-01-01), pages 353-366, XP019384715 ISSN: 1573-8965**
• **FARMANZAD ET AL.: "A novel model for biomechanical behaviour of human brain in epidural hematoma injuries" BIO-MEDICAL MATERIALS AND ENGINEERING, vol. 17, 2007, pages 119-125, XP009121930 cited in the application**

**Description**

**BACKGROUND OF THE INVENTION**

**Field of the Invention**

The present invention relates to non-invasive measurement and diagnostics of the Intracranial Pressure (ICP) for patients with brain injuries such as edema, hematoma or tumors.

**Background of the Invention**

There are a number of known measures for indicating the intracranial condition for patients with abnormal conditions due to brain injuries such as edema, hematoma or tumor:

- Intracranial pressure (ICP); the pressure underneath the cranium and which may be altered due to internal and external causes; today, only invasive methods are available for exact measuring ICP;
- Intraventicular gradient pressure (IGP); a measure of the difference between the pressures the ventricles in the two hemispheres;
- Displacement; showing how much a point in space has moved from its original position;
- Strain; a measurement of how much a certain volume has changed from its original configuration;
- Stress of the brain tissue; how much a certain force is acting on an area on an arbitrary plane in the brain tissue.
- Midline shift; the extent of midline shift is commonly used as a very generalizing measurement of likely increased ICP since the brain midline is pushed to the side as an abnormality (such as edema or hematoma) is growing. However, this is a very vague indication. Thus, in practice the measurements of main interest for diagnostics of brain damages are increased ICP and level of the patients' consciousness.

Traffic accidents are a major reason why patients are diagnosed or treated for brain injuries. In Sweden more than 20,000 patients with brain injuries caused by external violence were treated every year over the time period from 1987 to 2000. The major part (65%) of those injuries was represented by hematoma, diffuse brain injuries and edema where measurement of the ICP is crucial because elevated ICP can lead to hypertension and respiratory changes and can also contribute to damages in other areas of the central nervous system, outside the primary injury.

The size and location of the primary injury can be evaluated with high precision with radiological imaging such as Computer Tomography (CT). Traditionally, a CT scan is often performed when a patient with a head injury arrives to the emergency room. The doctor can then diagnose the severity of the injury based on the images. However, an estimation of the ICP of the patient is not provided by the images. To measure ICP, opening of the skull bone of the patient is necessary in order to insert a pressure sensor via a catheter. If the pressure is higher than a given level, the injury must be immediately evacuated to reduce the pressure to prevent further damages. On the other hand, if the pressure is below the critical threshold, conservative treatment such as intensive care can be used and further operations are not indicated.

The invention described herein is a non-invasive numerical method of measuring ICP, generally exemplified by a non-invasive Finite-Element method. When using the described invention the CT scan already at hand is used to perform a simulation of the hematoma or edema. The ICP of the patient can be measured non-invasively. Also local mechanical strains and stresses in the brain, which is related to subsequent brain injury, are measured. Stain and stress have not previously been used but the information is valuable for foreseeing possible complications and damages to the brain. Besides the gain of critical information, invasive operations are also avoided using this method, meaning less suffering and costs for the patient and the society.

**Description of the Prior Art**

There are a number of previously known non-invasive methods techniques to measure biological data within the brain. The closest related are:

- Magnetic Resonance Imaging of ICP measurement - the MRI-technique In this method MR-images are used to accurately estimate the in- and outflow to the intracranial cavity. These measured flows are then used in a flow model to estimate an elastance index, from which an ICP can be calculated. The method has been suggested but known clinical tests have so far been limited and only showed qualitative results. A study entitled *"Early Experience from the application of a noninvasive magnetic resonance imaging-based measurement of Intracranial pressure in Hydrocephalus"* by Roberta P. Glick et al presented November 2006 (Glick, R. P. et al, Neurosurgery: November 2006 - Volume 59 - Issue 5 - p 1052-1061) shows the application of the method. Notable is the fact that measures of flow are needed to calculate the ICP and the fact that it is only applicable for hydrocephalic patients (excessive amounts of cerebrospinal fluid). This method has not been tested with patients that have hematoma or edema. Also, this method cannot predict strain and stress that is in the affected brain. This method of using MRI scans is also more expensive than the herein described invention.
- Tissue resonance analysis - the TRA method In this method the mechanical responses of the intracranial tissues to the heartbeat and these responses' relations to elevations in ICP are exploited. The characteristic resonance response (eigenfrequency) of the third ventricle walls is recorded in an echopulsogram and empirically related to ICP by a simple formula. The method is based on changes caused by the changing shape of the ventricular wall during a cardiac cycle. A study on this method is presented in "Tissue resonance analysis: a novel method for non-invasive monitoring of intracranial pressure" found in J Neurosurgery 96:1132-1137, 2002. Tests show good correlation with invasive measurements. However, the method is dependent on measurements over time giving the changes in ICP based on heart rhythm and thus flow of blood. Furthermore, this method cannot predict strain and stress that is in the affected brain.

• Transcranial Doppler ultrasonography (TCD) - a system analysis approach This method consists of relating the flow characteristics of the arterial blood flow to the ICP. Such a relation has been established, and by assuming a system analysis approach, a method of non-invasive estimation using TCD for blood flow measurements is developed. The method offers monitoring possibilities and the reconstruction of the ICP wave for further analysis. A method of using TCD to measure ICP among others is disclosed in U.S. Patent No. 6875176 and in "Transcranial Doppler sonography pulsatility index (PI) reflects intralranial pressure (ICP) " by Johan Bellner et al. (Surgical Neurology, Volume 62, Issue 1, Pages 45-51) The method is however depending on the operator and the angle of insonation and is unable to measure strain, stress and pressure that can vary in the brain.

The three above mentioned methods are based on information based on the flow of blood or CSF. In some cases the information is combined with spatial information from medical images but measurement cannot be obtained solely from the spatial information given from a medical image. Furthermore these methods cannot predict ICP, stain and stress of the patient's brain, which is useful in order to give a full understanding of the condition. Therefore they differ substantially from the spatially based numerical methods thought in the invention disclosed herein.

A number of known numerical methods are presented below and relating previous studies using FEM are discussed.

• Finite difference methods (FDM)

Like the finite element method, this method is a numerical method used to solve partial differential equations. The difference between the two methods is that FDM is an approximation to the differential equation while FEM is an approximation to its solution. FDM is easy to implement but less flexible in the ability to handle complex geometry.

• Finite volume method (FVM)

This is a method similar to FDM and calculates conserved variables, e.g. fluxes entering and leaving a finite volume using the divergence theorem. The difference is that FVM does not require a structured mesh as in FDM. It is often used in computational fluid dynamics (CFD).

• Meshless method

Previously mentioned methods requiring a mesh to discretize the differential equations and complex geometry will sometimes lead to difficulties in the mesh generation. By formulating the discretization with a meshless approach, the problem associated with meshing can be avoided.

• Finite element methods (FEM)

The finite element method has long been used in space and aero industry to calculate mechanical entities such as strain, stress and pressure in their construction. The finite element method was developed mainly during the 1960's and 1970's. During the past decades, development of small powerful personal computers and workstations has made FE-codes a tool as common to many engineers as the pocket calculator. It has also become much more accessible through the easy-to-use interfaces provided by most commercial FE-codes.
The basic principles of FEM are dividing a complex numerical problem (a structural system) into manageable problems (finite elements) and the solution of the complex problem can be achieved. Each element in the structural system is modeled with the corresponding physical properties. The purpose of such numerical modeling in structural and fluid mechanics is to predict the response of mechanical systems that are exposed to specific loads or initial conditions. This is achieved by: 1) formulating a set of equations that realistically describe the physics of the system, and 2) solving these equations with appropriate boundary conditions.
Studying biomechanics using the finite element method has been ongoing in the past century. The biomechanics of the human head can be seen as a brain movement within an externally loaded skull and this gives a complex three-dimensional dynamic boundary value problem. These internal biomechanical responses of the brain cannot be completely measured by experimental techniques. Analytical models are limited to problems with relatively regular geometry, simple boundary conditions and homogeneous material properties. Numerical approaches, on the other hand, approximate the analytical solution with a numerical procedure. The finite element method is superior to other numerical methods when it comes to modeling of irregular geometry, inhomogeneous and nonlinear material properties and complex boundary and loading conditions. Finite element models with human anthropometry have been developed through the years that can predict injury with good accuracy. Using the finite element method to study clinical pathology related to biomechanics is a relative new area and preliminary studies indicate that the results are useful in clinical diagnoses.

Head injuries are related to tissue material failure, characterized in some form of stress, strain or deformation. Numerical methods such as finite element analysis can therefore provide stress, strain and deformation distributions across and within the different tissues for a given biomechanical input, such as head motion or head impact. By identifying the magnitudes and location of these quantities, at which the tolerance level of the tissue is exceeded, provides the link between the external mechanical quantities and the internal injuries. Finite element models are repeatable and reproducible, and simulations can be seen as surrogate experiments without biological variability. Such a model of the human head makes it easier to understand what happens in a human head during an impact.

One method of using a finite element model in an intra-operative setting is described in U.S. Patent No. 7,072,705 *"Apparatus and methods of brain shift compensation and applications of the same"* claiming to find out the intra-operative brain shift by solving equations with the finite element method (using a finite element model). This is used in image guided surgery and in the image the position of the brain is compensated / shifted from the pre-operative image to a calculated one. However ICP measurement is not mentioned nor is it used for diagnostic or assessment of the condition of the brain.

Finite element models have been presented evaluating biological measurements. Farmanzad et al. (Bio-Medical Materials and engineering 17 (2007) 119-125) discusses the use of finite element model for analyzing biomechanical behavior in the human brain during a case of epidural hematoma. A two dimensional finite element model was constructed based on the CT scan of a 31-year-old male patient who suffered from hematoma. The authors conducted a parameter study on different elastic module (E), poisson ratio (v) and intra ventricular pressure gradient ($\Delta P$) and compared the ventricular shapes of the model and the patient. The authors concluded two criteria for E and $\Delta P$. These parameters were used to optimize the model. Other known solution applying FEM to evaluate the intra-ventricular pressure gradient was Saberi et al. (Computer Aided Surgery, Volume 12, Issue 2 March 2007, pages 131 - 136). As Farmanzad et al. (2007), a CT image of a patient suffering from epidural hematoma comparing the displacement of the reference points of the ventricle with the ventricle in the patient. However, the two above mentioned studies describe neither intracranial pressure nor using strain or stress in the model. Furthermore, the studies are single cases for evaluating a mathematical model in a fixed setting, not as in the invention herein providing a simulation or as a diagnostic tool.

Further, M. Shill et al. suggest a finite element model to determine the maximum displacement of the falx cerebri (Biomechanical Simulation of the Falx cerebri using the Finite Element Method (1996), M. Schill, M. Schinkman, H.-J. Bender, R. Manner). Measurement of ICP in the two hemispheres is used as input data for the calculation of displacement. However, the method is neither based on medical images nor on patient-specific data.

Cheng et al. discusses another finite element model that simulates midline shift during hematoma (The correlation of midline shifts of human brain with large brain haematoma using a finite element approach, Cheng AY, Paun MC, Poon WS, Wong GK). A 5mm thick FE-model was created based on CT and MRI scan images of a patient with hematoma. The model was then used to simulate the hematoma in different locations in the brain in order to quantify the maximum displacement of the midline shift in the brain. The authors found that prediction in lobar situated hematoma cases was more accurate than the deep-seated ones and that there is a linear relation between the size of the lesion and the maximum displacement of the midline shift. However, the model is not three-dimensional but based on a horizontal slice of a head. When analyzing midline shift located on the same plane as the hematoma, it might be sufficient with a two-dimensional mode but, as Cheng et al. teaches, a three-dimensional model is more accurate in cases with lobar hematoma. Cheng et al. evaluates the midline shift but as in the invention herein the intracranial pressure was not measured using the model and neither did they look at the maximum principal strain and stresses in the brain.

A complete FE-model of the head and neck has been developed at the royal institute of technology in Stockholm by Svein Kleiven et al. (hereinafter called "KTH model"). The KTH model has implemented more complex material models and is more extensively validated than other models and correlation was found between the injury pattern found in CT images of a patient being the victim of a motocross accident and the strain pattern found in the model in the reconstruction using the KTH model. However, the FE-model is not used to predict ICP or strain after an injury has occurred. The model is described by Kleiven in "Predictors for traumatic brain injuries evaluated through accident reconstructions " 51st Stapp Car Crash Journal, 2007). The article describes a method to compare ICP with the temporary stress during an external impact of the head since the dynamic movement in the brain can cause injuries. The KTH model has throughout its development been based on the same geometry but with varying material parameters. The invention described herein teaches an improved FE-model and a non-invasive brain injury evaluation used when an injury has occurred and static changes in the brain (such as hematoma and edema) have occurred.

The basic parameters of the previously known KTH FE-model are furthermore validated against cadaver experimental data for different impact directions. Kleiven argues in "Correlation of an FE-model of the Human Head with Local Brain Motion-Consequences for Injury Prediction" (46th Stapp Car Crash Journal, 2002) that values of the shear properties of the human brain should be lowered in most existing FE-models. The available FE-model has therefore validated basic stiffness parameters and tissue properties for a situation to predict a localized brain response of a temporary external impact to the human head.

T.J. Horgan and M.D. Gilshirst have developed and validated another FE-model (se for example "Influence of FE model variability in predicting brain motion and intracranial pressure changes in head impact simulations" in International Journal of Crashworthiness 2004, vol 9 No.4 pp. 401-408). Aspects of designing an FE model are discussed but, in the same manner as the KTH FE-model, only temporal and external impact is discussed.

The above mentioned methods are developed to measure ICP but not strain and stress efficiently. Robert W.G. Anderson has, however, discussed the relation between ICP and stress (Anderson, R.W.G., Brown, C.J., Blumbergs, P.C., Scott, G., Finney, J.W., Jones, N.R., and McLean, A.J. (1999). Mechanics of axonal injury: An experimental and numerical study of a sheep model of head impact, Proc. 1999 IRCOBI Conf. Sitges, Spain, pps. 107-120. Injury. Journal of Biomechanical Engineering 16, pp. 615-622.).

In these respects, analyzing ICP and/or stress and strain after an injury using a numerical method based on spatial information from medical images according to the present invention substantially departs from the conventional concepts and designs of the prior art.

## SUMMARY OF THE INVENTION

The general purpose of the present invention, which will be described subsequently in greater detail, is to provide a non-invasive numerical method for measuring Intracranial Pressure (ICP), strain and stress for patients who suffer from hematoma or edema. A patient-specific three-dimensional finite element model with natural biomechanical response is used. Medical images such as CT or MR images are used to create a patient-specific FE-model in order to give an individual diagnosis and treatment plan for each patient. Thus, the present invention gives a new, complementing analysis method of medical images giving qualitative information.

A primary object of the present invention is to provide for a non-invasive method for Intracranial Pressure (ICP) measurement based on medical images and a numerical method.

An object of the invention is to provide a patient-specific three-dimensional numerical model for non-invasive ICP measurement.

Another object of the invention is to provide a patient-specific three-dimensional finite element method for non-invasive ICP measurement.

An object of the present invention is to provide a novel numerical model simulating the natural biomechanical response of the human brain.

Another object is to provide for complementing methods of analyzing the injuries in human head using strain and stress as parameters of the numerical model.

A further object of the invention is to provide, based on the novel patient-specific numerical model and methods of analyzing injuries in the human head, for a probability of further injuries in the brain and probable results and needs for invasive measures and/or treatments.

Other objects and advantages of the present invention will become obvious to the reader and it is intended that these objects and advantages are within the scope of the present invention.

## BRIEF DESCRIPTION OF THE DRAWINGS

Figure 1 shows the basic method of the invention using an FE-model.
Figure 2 shows the basic method of the invention using an auto generator.
Figure 3 in the left image shows the patient's bleeding in white and how it compresses the brain tissue. The right image shows the numerical estimation of the brain deformation illustrated with in grayscale.
Figure 4 shows the ventricles in the original FE-model (left). As can be seen, there is no communicating channel between the third and the fourth ventricle, as for the true anatomy (right).
Figure 5 shows the new ventricular system with the constructed communicating channel.
Figure 6 is a schematic figure showing a cross-section of the three-dimensional model. Nodes on the brain surface are moved to simulate the epidural hematoma. The fringe levels indicate the level of strains from 0 to 0.3.

## DETAILED DESCRIPTION OF THE INVENTION AND PREFERRED EMBODIMENTS THEREOF

The present invention is a non-invasive numerical method for measuring intracranial pressure (ICP), strain and stress in brain tissue for a patient who suffers from hematoma, edema or tumor. To attain an accurate result in the numerical method, a patient-specific three-dimensional finite element model is used. Medical images such as CT or MR images from the patient are used to generate the patient-specific FE-model. The finite element model simulates the complications that the patient suffers from, providing valuable information for individual diagnosis and treatment. Thus, the present invention gives a new, complementing analysis method of medical images giving qualitative information.

The novel tool described herein relates to a patient-specific diagnosis in the health care system. The new diagnosis method is designed to complement currently used radiological imaging techniques by adding information about the mechanical loads that the injured and surrounding tissues experience. The unique method will improve and increase efficiency of patient examinations for the radiologists and neurosurgeons and will improve diagnoses so that treatment can be optimized for each specific patient and injury. The quality of treatment is improved as well as reducing treatment costs and human suffering.

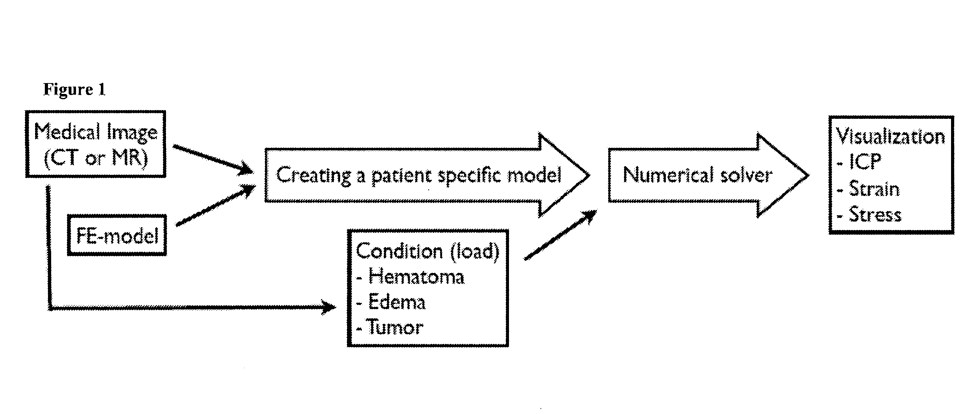

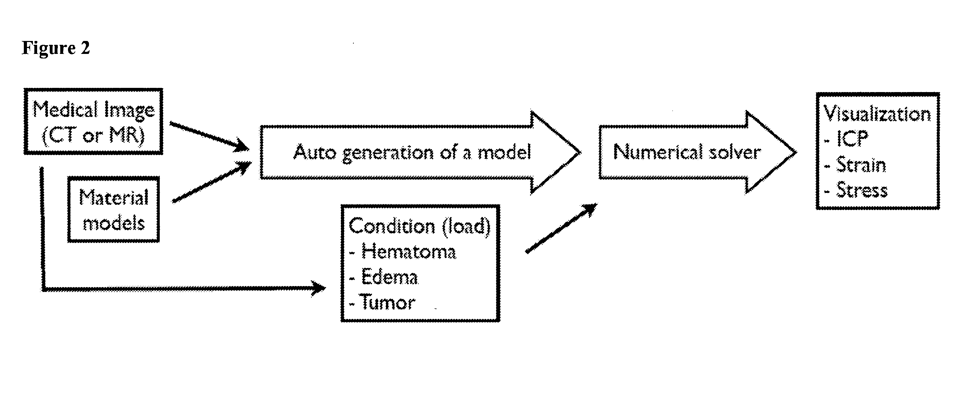

The method used in the invention described herein is generally a numerical method and specifically a FE-model to calculate ICP and other biological data such as strain and stress of the patient's brain. The method is patient-specific and based on medical images such as CT or MR. The method can either be based on a patient's medical images and a numerical model (Figure 1) or a patient's medical images and an auto generation of a model (Figure 2). The first method using a numerical model is more efficient and demands less computational power and the second auto generating method is presumably more specific.

Independently of the chosen method the invention herein is characterized by being three dimensional and patient-specific. The method is based on a model (such as an FE model) corresponding to the natural biomechanical response of the brain. Some aspects of the invention are described more in detail below.

### Patient-specific

There are two ways of making the FE-model patient-specific. Either a new model can be generated or an existing model can be morphed to fit the anthropometry of the patient. To generate a new model, a three-dimensional MR image of the patient is needed. The different tissues in the head are classified using image processing algorithms, e.g., an estimation maximization classification method. This method in particular is robust and is able to produce good result even with the presence of an inhomogenity field that is common in MR images. An inhomogenity field has the effect of making some parts of the image brighter than they should be, hence making it more difficult to classify different tissues correctly. The different tissues are then converted to an FE-model by turning each picture element into a fmite element with a volume that corresponds to the spacing of the three-dimensional image. A smoothing of the surface nodes is then performed to decrease the numerical error due to the unsmoothed surface.

To morph an existing model into the shape of the patient, the primary input data is a three-dimensional CT-scan image. First, the brain is segmented from the picture using a segmentation algorithm such as level set segmentation and the result is a binary image that depicts the brain in bright intensity and background as dark intensity. The brain of the existing FE-model is also converted to a binary image which is used to create a deformation map for the whole FE-model. An image registration is performed to create the deformation map, which is to find a spatial transform mapping one image into another. Using the deformation map, the nodes of the existing FE-model are transformed spatially and the resulting model should have a shape related to the anthropometry of the patient.

In order to attain an accurate ICP and other biological data for every individual case, the patient-specific FE-model simulates the natural biomechanical responses in the human brain. In other words, the model acts in the same manner as the specific patient's brain considering the stiffness and elastance and other factors in each element of the model.

There are some aspects to consider and to include in the FE-model used in the invention described herein.

### Natural biomechanical response

Besides having the right geometry, correct material properties in the FE-model are important for a simulation corresponding to the natural biomechanical response in the human brain. To make the FE-model "biofidelic" (containing correct material properties), serious parameter studies of the different materials are validated against experimental data such as brain relative movement to the skull in impact or pressure pattern that are generated in an impact. With higher correlation to the experimental data, the higher biofidelity it is for the FE-model, therefore the result from the simulation is more reliable.

When developing a FE-model of the brain, it is beneficial if the model can mimic the compliance of the central nervous system. Therefore remodeling of the flowing properties of the ventricular cerebrospinal fluid (CSF) is one aspect of the invention. An example of communicating ventricular system is described in example 1. Therein, simulation of an Eulerian formulation of the ventricles and their communicating channel shown and implemented to model the flow of CSF correctly and aspects to be considered when calculating intracranial pressure in an FE-model are shown.

An alternative to modeling the communicating ventricular system is to create an FE model where the material properties of the models (presumably the bulk modulus of the CSF) are altered to mimic the compliance that is otherwise simulated by the communicating ventricular system.

Measurement of ICP in the invention described herein can be complemented by other measurements to give an improved criterion for injuries. Strain can be used as a novel criterion for the effect and influence of diseases in living tissues. Changes in strain in anatomical and histological structures occur due to inner and outer effects on the tissue. The strain is to be compared to the normal structure and calculated as a change in percentage in relation to the normal structure. The level of strain has been shown to correlate with injuries. Since physiological changes associated with injures do not always occur immediately, measurement of strain gives the possibility of predicting injuries such as diffuse axonal injury, contusion and hemorrhage. Stress can be used as a complement or alternative to ICP and strain. The relation between forms of stress (von Miese stress) and Diffuse Axonal Injury (DAI) but not the clinical application has not previously been shown. Thus, when information on strains and stresses is available, the medical team has the possibility of preparing treatments before the physiological changes in the patient's brain occur.

The invention described herein is a useful tool not only to calculate ICP, strain and stress but also as a diagnostic tool giving the probability of secondary injuries in different regions of the patient's brain. Furthermore the invention described herein can be applied in a setting giving suggestions for treatments and probable consequences in the patient's brain of those treatments. Below is an illustration of a typical clinical application of the invention described herein. In example 2, an example of calculating ICP with a FE-method is shown.

**Clinical Application of Non-Invasive Brain Injury Evaluation**

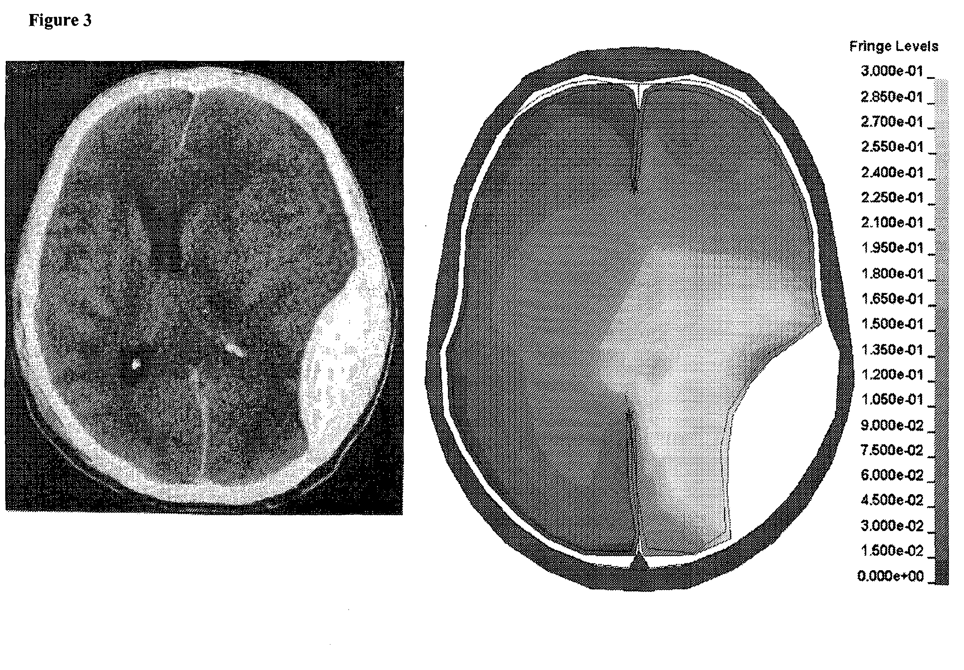

A patient who has experienced a traumatic accident is admitted to the hospital and shows symptoms of brain injury. The patient is examined at the radiology department using medical imaging techniques, such as CT (Computer Tomography) or MRI (Magnetic Resonance Imaging). Based on these images an FE-model of the skull and brain is scaled to accurately represent the specific anatomy of this patient. The next step is to simulate the brain injury in the FE-model based on the volume and degree of injury visible on the medical images. The results from the FE simulations provide unambiguous information of the intracranial pressure in the numerical brain. This numerical pressure is a good estimation of the actual pressure that the patient's brain is experiencing, illustrated in Figure 3 for an intracranial hematoma. When the estimated pressure is below the critical value an unnecessary surgical intervention has been avoided. On the other hand, if the estimated pressure is above the critical value the neurosurgeon has more available information to evaluate how the central nervous system is affected by the injury and the state of the patient. Therefore, using the tool described herein it is possible to analyze the brain pressure or the intracranial pressure based on numerical methods rather than surgical interventions. In Figure 3 a basic simulation is shown. The left image shows the patient's bleeding in white and how it compresses the brain tissue. The right image shows the numerical estimation of the brain deformation illustrated with a grey scale, in the KTH head model© simulating the response due to an intra-cranial bleeding with similar volume and location.

As to a further discussion of the manner of usage and operation of the present invention, the same should be apparent from the above description. Accordingly, no further discussion relating to the manner of usage and operation will be provided.

Therefore, the foregoing is considered as illustrative only of the principles of the invention. Further, since numerous modifications and changes will readily occur to those skilled in the art, it is not desired to limit the invention to the exact construction and operation shown and described, and accordingly, all suitable modifications and equivalents may be resorted to, falling within the scope of the invention.

**EXAMPLE 1- communicating ventricular system**

Construction of the aqueduct of Sylvius and a simulated outflow from the fourth ventricle

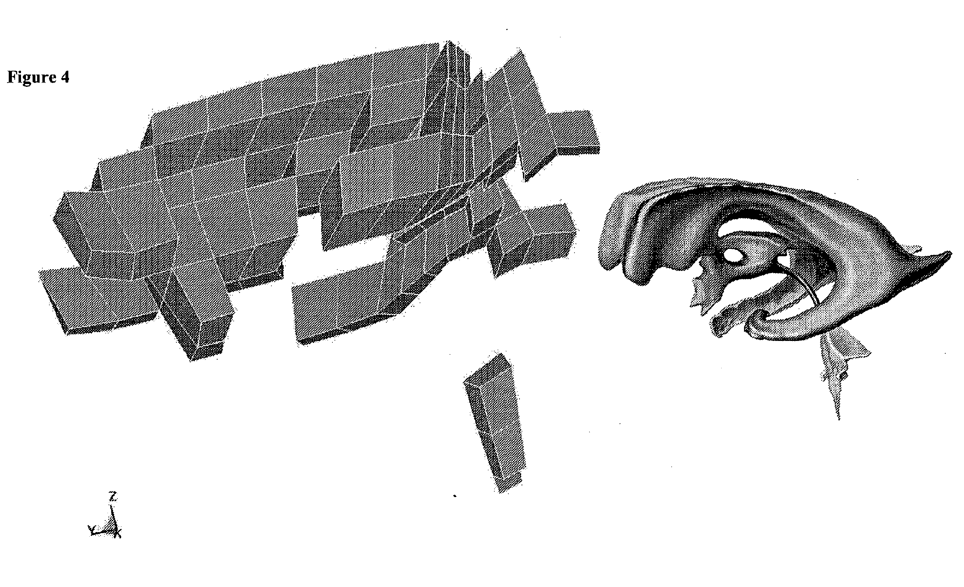

When developing a FE-model of the brain it is beneficial if the model can mimic the brain compliance. Therefore remodelling of the flowing properties of the ventricular cerebrospinal fluid (CSF) is one aspect of the invention. The CSF makes up a circulatory system in the intracranial space. CSF is formed deep within the brain in the ventricles, from where it then flows out into the subarachnoid space and finally drains into the sinuses and follows the venous blood out of the skull. The compliance function of the brain is dependent on the existence of such communication so that CSF can be evacuated from the intracranial space in the presence of an expanding mass lesion. In the original "KTH head" model, no interventricular communication existed. In order to mimic any brain compliance, communicating channels in the ventricular system had to be constructed, or more specifically the communicating channel between the lateral and fourth ventricles, the so-called aqueduct of Sylvius. See Figure 4 for the ventricles in the original FE-model (left). As can be seen, there is no communicating channel between the third and the fourth ventricle, as for the true anatomy (right).

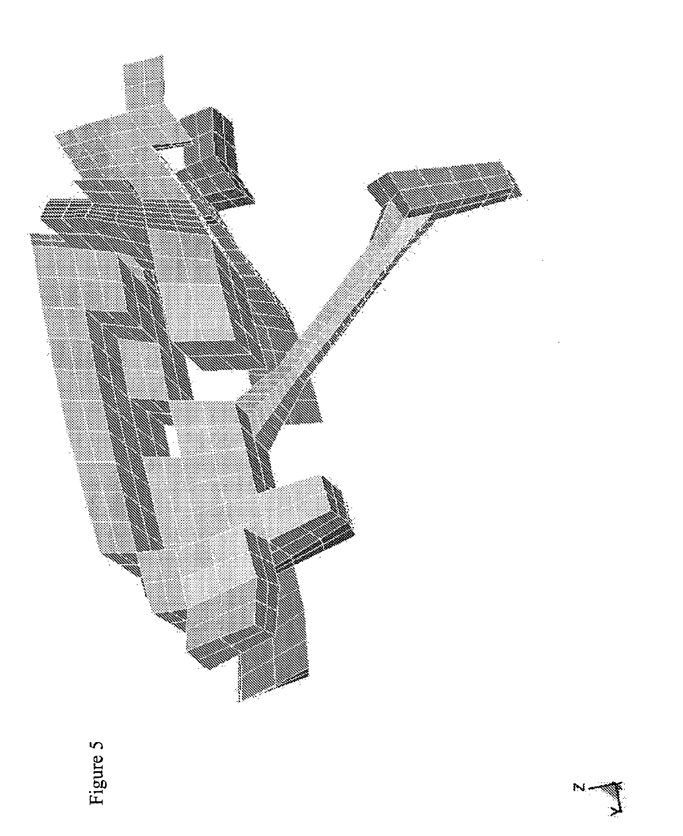

The aqueduct of Sylvius was modeled by creating an outflow in the third ventricle and an inflow in the fourth ventricle, and then connecting them by a channel (Figure 5). First the ventricle elements were split, and then enveloped by a thin elastic shell with common surface nodes. The in- and outflows were then constructed by deleting shell elements at the location of the holes. A channel of elements adjoining the exposed ventricle elements in the in- and outflows was then created. The elements in the channel were constructed so that they would have the same characteristic lengths as the ventricular elements to which they were connected at the in- and outflow. The channel was also enveloped by a thin elastic shell, which was merged at the in- and outflows to the shells covering the ventricles. To be able to study flows in the ventricles, the multi-material formulation in LS-DYNA™ was used. For this the lateral ventricles were defined as two parts, the channel as a third and the fourth ventricles as a last fourth.

However, the modeled channel could not be connected to any surrounding structures in the model, except the in- and outflows in the ventricles. Therefore movements in the surrounding structures will not influence the model. This deficiency does not change its function as a communicating channel between the third and fourth ventricles, as it will still transport CSF according to the pressure gradient along the channel. See Figure 5 for the new ventricular system with the constructed communicating channel. The evacuation of CSF from the ventricular system is modeled by a hole in the lower part of the fourth ventricle. An uncoupling of the Eulerian mesh and the surrounding elastic shell simulates the hole. Nothing will then hinder the CSF from "flowing" out of the hole, and an evacuation of CSF out of the intracranial space is thus simulated.

Eulerian formulation of CSF

A Eulerian formulation of the ventricles and their communicating channel is implemented to model CSF correctly as a fluid. The Euler mesh was in this case constructed by first splitting the original Lagrangian ventricle mesh, then reformulating the element as Euler elements and finally construct a thin elastic shell enveloping the Euler ventricles and having nodes in common. The shell is also linked to the surrounding Lagrangian structures, thus coupling the Eulerian ventricles to the Lagrangian brain.

The CSF is modeled as an elastic fluid by the material card *MAT_ELASTIC_FLUID, with a density $\rho$ of 1 kg/dm$^3$, a bulk modulus $K$ of 2.1 GPa and a tensor viscosity coefficient of 0.3. No hourglass control should be used for fluids since no zero energy modes exist. Due to a possible bug, LS-DYNA™ assigned a non-zero hourglass coefficient to elements predefined to be exempted from hourglassing. Because of this the ventricular elements have been assigned a hourglassing with a very low hourglass coefficient ($10^{-10}$).

The elastic shell has been defined as a viscoelastic material by the card *MAT_GENERAL_VISCOELASTIC. The shell has a density $\rho$ of 1.040 kg/dm$^3$, a bulk modulus K of 210 MPa. Its viscoelastic properties resemble those of the meninges. The elastic part is defined in the range 52 - 5200 kPa, dependent on different parts of the elastic shell. For example, the shells surrounding the aqueduct of Sylvius and the fourth ventricle were too weak in the first simulations, resulting in decreased time step due to deformation of shells and subsequent distortion of the underlying Eulerian elements. Therefore, these shells were stiffened to reduce deformation and resulting computation costs.

Calculation of ventricular pressure

The ICP is calculated as the mean pressure of elements in the lateral and third ventricles, both in the case for the channel model and the old "KTH model". Physically, the pressure in the CSF is the sum of a positive static pressure and a varying dynamic pressure. The static pressure is a reference pressure normally taken as the mean pressure in the container, and the dynamic pressure equals the element deviation from this static pressure. In the clinical situation, the static pressure is measured. However, in the simulations the SDH [subdural hematoma] is reconstructed dynamically and because of this the dynamic component of the element pressure can, and proved, to be significant in the individual elements. Nevertheless, it was not obvious which elements should or could be used for the mean value calculation of the static pressure. One reason for this was that the elements adjacent to the Lagrangian structures for which the fluid/structural influence from ventricular walls were difficult to appreciate, i.e. it was not known if the pressure in the Eulerian elements adjacent to the Lagrangian surrounding structures would be a physically correct pressure. However, this was also the case for the old model, in which the element pressure also showed of great dynamic variation. All the same, analysis of different element pressure showed that the best procedure for retrieving a "stable" and physical plausible ICP-measure for both models was to use the mean pressure of all elements in the lateral and third ventricles.

**EXAMPLE 2- the principles of the FEM-analysis of the brain**

The principle outlined is of the disclosed invention with measuring ICP (IntraCranial Pressure) and strain due to hematoma inside the skull. When admitting a patient to the hospital, who has experienced a traumatic accident, examination at the radiology department will be carried out using CT (Computer Tomography). In cases where bleeding is occurred inside the skull, an assessment of ICP is necessary in deciding whether the patient should be operated on or not. Using the disclosed invention which is a non-invasive method, unnecessary incision is avoided, saving money for the hospital and minimizing suffering for the patient.

The material used in the example is:

    a. three-dimensional CT images of the patient's head.
    b. a computer with finite element solver.

A finite element solver is computer software that calculates the approximated solutions to the partial differential equations and in this case specifically analyzes structural problems. The solver requires as input a description of the geometry, boundary conditions and loading conditions of the problem. The source code of such software is publicly available as a freeware and is also commercially available. In this particular example, the finite element solver used is a commercial version called LS-DYNA™ (available from Engineering Research, Linköping; Sweden)

In the particular example described below, the material properties for brain tissue is a second order Ogden hyperelastic constitutive model and corresponding parameters was fitted using discrete spectrum approximation (described in the "KTH head") as described by Puso and Weiss (J Biomech Eng. 1998 Feb; 120(1): 62-70) to include the non-linear elasticity described by Franceschini (The mechanics of human brain tissue, PhD-thesis 2006, University of Trento, Italy) and Franceschini et al. (J Mechanics and Physics of Solids,2006, 54(12): 2592-2620.) as well as the high frequency relaxation moduli determined by Nicolle et al. (2005 Biorheology, 42(3): 209-23).

Using the relationship $G=\frac{1}{2}\Sigma \alpha_i . \mu_i$ for the Ogden parameters gives an effective longterm shear modulus of around 1 kPa. These parameters derived from the experimental work of Franceschini (2006) give a quasi-static stiffness for the brain tissue that is around the average published experimental values (Donnelly, 1998, A comparison of results. Biomechanical research: Experimental and computational, Proc. Of the 26th int. Workshop., pp. 47-57). The values used in this example are: $\mu_1$ = 53.8 Pa, $\mu_2$ = - 120.4 Pa; $\alpha_1$ = 10.1, $\alpha_2$ = -12.9; $G_1$ -$G_6$ (kPa) = 320, 78, 6.2, 8.0, 0.1, 3.0 and $\beta_1$ - $\beta_6$ = $10^6$, $10^5$, $10^4$, $10^3$, $10^2$, $10^1$. The following Ogden constants were determined for the brain stem: $\mu_1$ = 15.8 Pa, $\mu_2$= -106.8 Pa, $\alpha_1$ = 28.1 and $\alpha_2$ = -29.5. The relaxation moduli were assumed to be 60 % higher than those for the gray matter in the cortex (Arbogast and Margulies, 1997, Paper No. 973336, Society of Automotive Engineers, Warrendale, PA); and Franceschini, (2006).

When performing a FEM analysis of hematoma inside the skull, the KTH-model is adjusted to the specific anatomy of the patient based on the three-dimensional CT images. In this particular example, the adjustment to the anatomy of the patient is done with registration method provided by the Insight Segmentation and Registration Toolkit (ITK) (available from Kitware, Inc., New York 12065, USA)The brain in the KTH-model is first converted to a binary image which is served together with the patient's CT images as input in the registration method. This results in a deformation field that can be used to transform the coordination of the nodes of the KTH-model.

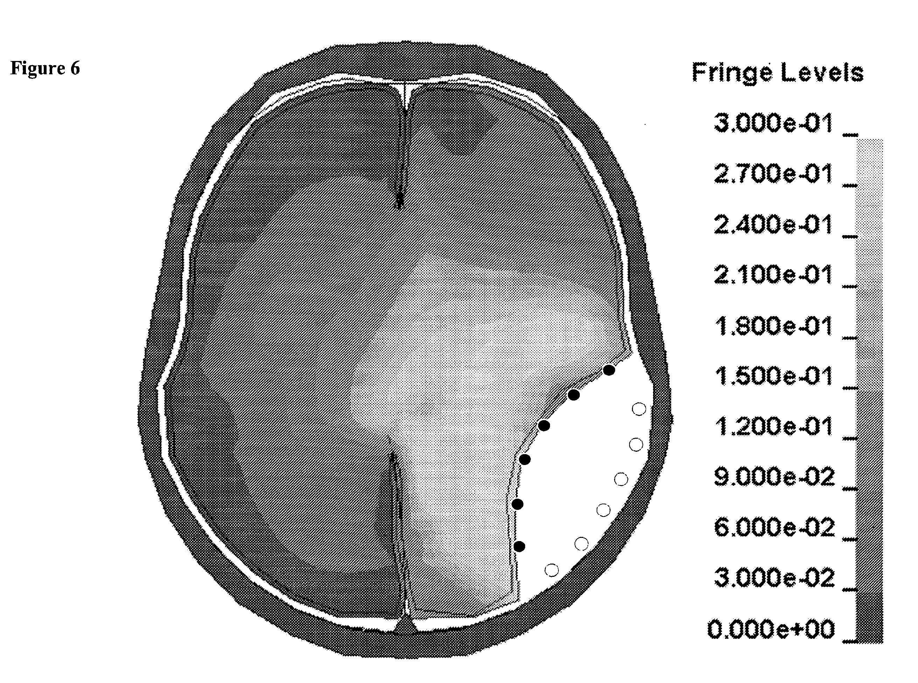

Using the segmentation method provided by the ITK, the hematoma is segmented and is reconstructed by displacing nodes on the cortex within prescribed motion. The natural tissue motion would be in the normal surface direction, since the displacement is caused by pressure from the haematoma (Figure 6). Since some areas are more indented than others in a hematoma, the displacement will be different along the surface. Moving the nodes of the elements brings about the motion of the surface. However, the normal direction of the nodes is not available as input motion direction in LS-DYNA™ (actually because the normal is simply not attainable). Only x-, y- and z-displacements are user-definable. Therefore the main displacement has been defined to be along a vector approximately normal to the cortex surface. Many simulations were performed to render a set of nodal displacements for which the relative shapes of the elements are maintained. This will give a good surface shape and also guarantee computing stability. The current position of a node with node number $N_i$ is set to $\mathbf{x}^{N_i}(t)$ and is calculated as

$$ \mathbf{x}^{N_i}(t) = \mathbf{x}_0^{N_i} + h \cdot \mathbf{x}_{displ} \cdot \frac{t}{T} . $$

This means a linear displacement in time along a vector $\mathbf{x}_{dixpl}$ scaled by a factor h. $\mathbf{x}_0^{N_i}$ represents the nodal position at $t$ = 0, and $T$ the termination time. In this way the node is displaced a distance $h$ along the vector $\mathbf{x}_{displ}$.

The FE-solver will then solve this structural problem and presents the final result as ICP and/or strain. The governing equations for structural problems (given by LS-DYNA™) are 1) conservation of mass; 2) conservation of momentum; 3) conservation of energy; 4) strain-displacement equation; and 5) constitutive equation. These governing equations can be expressed in one single governing equation by substituting into the momentum equation used in the conservation of momentum. This momentum equation and the traction boundary conditions are used to form the principle of virtual work which is the discretization of the structural problem that can be solved by the finite element method. In general, after each time step in the simulation, the nodal displacements of the mesh are calculated and strain and stress in each element can be derived from that. The pressure of each element is in its turn derived from the stress. The intracranial pressure of interest is the average pressure of the brain tissue in the finite element model. Depending on the level of ICP and more importantly the strain, the doctor can decide a suitable treatment for the patient.

**Claims**

1. A non-invasive method of evaluation of a patient brain, comprising:

   a) obtaining medical images of the patient brain;
   b) forming a patient-specific three-dimensional model of the patient skull and brain using the medical images and a numeric model;
   c) simulating brain injury in the patient-specific three-dimensional model based on the volume and degree of injury visible on the medical images; and

d) using the result of brain injury simulation in the three-dimensional model to provide information on the patient intracranial pressure.

2. The method of claim 1, wherein three-dimensional model is made patient-specific by generating a new model of the patient brain using a three-dimensional magnetic resonance image of the patient brain.

3. The method of claim 1, wherein the three-dimensional model is auto-generated.

4. The method of claim 3, wherein the three-dimensional model is auto-generated by:

a) obtaining a three-dimensional image of different brain tissues of the patient brain using magnetic resonance imaging;

b) converting the three-dimensional image to a finite element model by turning each image element into a finite element with a volume corresponding to spacing of the three-dimensional image;

c) smoothing surface nodes on the finite element model to decrease numerical error and form the three-dimensional model.

5. The method of claim 1, wherein the three-dimensional model is generated by changing an existing model to fit patient anthropometry.

6. The method of claim 5, wherein the three-dimensional model is generated by

a) obtaining a three-dimensional image of the patient head using a three-dimensional computer tomography scan image;

b) using a segmentation algorithm to segment the three-dimensional image to form a binary image of the brain;

c) using the binary image and an existing finite element model, converted to a binary image, to create a deformation map for the finite elements model; using the deformation map to dislocate the nodes in the existing finite element model to fit the anthropometry of the patient.

7. The method of claim 1, wherein the tissues in the head of the patient are classified using image processing algorithms.

8. The method of claim 1, wherein obtaining the three-dimensional model comprises remodeling the flowing properties of the ventricular cerebrospinal fluid of the patient utilizing simulation of an Eulerian formulation of the patient ventricles and the communicating channel of the ventricles.

9. The method of claim 1, wherein obtaining the three-dimensional model comprises creating a finite element model where the bulk modulus of the cerebrospinal fluid is altered to mimic compliance of the central nervous system.

10. The method of claim 1, further comprising measuring strains and stresses in the brain, and utilizing the measured strains and stresses to foresee possible complications and damages to the brain.

11. The method of claim 1, further comprising obtaining measurements of strain in anatomical and histological structures, and comparing the measurements to normal structure to correlate with injuries to the patient.

12. The method of claim 1, wherein the patient suffers from hematoma, edema or tumor.

13. The method of claim 1, wherein a computer with finite element solver software is used in a method of noninvasive measurement and diagnostics of the intracranial condition for patients with abnormal conditions due to brain injuries .

14. The method of claim 1, further comprising determining the probability of further injuries in the brain and probable results and needs for invasive measures and/or treatments.

**Patentansprüche**

1. Nicht-invasives Verfahren zur Untersuchung des Gehirns eines Patienten, umfassend:

a) Gewinnen medizinischer Bilder des Gehirns des Patienten,

b) Erstellen eines patientenspezifischen dreidimensionalen Modells des Schädels und Gehirns des Patienten unter Verwendung der medizinischen Bilder und eines numerischen Modells,

c) Simulieren einer Gehirnverletzung in dem patientenspezifischen dreidimensionalen Modell anhand des auf den medizinischen Bildern sichtbaren Umfangs und Grads der Verletzung, und

d) Verwenden des Ergebnisses der Simulation der Gehirnverletzung in dem dreidimensionalen Modell zur Bereitstellung von Informationen über den Hirndruck des Patienten.

2. Verfahren nach Anspruch 1, wobei das dreidimensionale Modell durch das Erzeugen eines neuen Modells des Gehirns des Patienten unter Verwendung eines dreidimensionalen magnetresonanztomographischen Bilds des Gehirns des Patienten patientenspezifisch gestaltet wird.

3. Verfahren nach Anspruch 1, wobei das dreidimensionale Modell automatisch erzeugt wird.

4. Verfahren nach Anspruch 3, wobei das dreidimensionale Modell automatisch erzeugt wird durch:

a) Gewinnen eines dreidimensionalen Bilds von unterschiedlichen Hirngeweben des Gehirns des Patienten unter Verwendung der Magnetresonanztomographie,
b) Umwandeln des dreidimensionalen Bilds in ein Finite-Elemente-Modell durch Verwandeln jedes Bildelements in ein finites Element mit einem Volumen, das dem Abstand des dreidimensionalen Bilds entspricht,
c) Glätten von Oberflächenknoten in dem Finite-Elemente-Modell zur Verringerung des numerischen Fehlers und zur Erstellung des dreidimensionalen Modells.

5. Verfahren nach Anspruch 1, wobei das dreidimensionale Modell erzeugt wird, indem ein bestehendes Modell verändert wird, um es an die Patientenanthropometrie anzupassen.

6. Verfahren nach Anspruch 5, wobei das dreidimensionale Modell erzeugt wird durch

a) Gewinnen eines dreidimensionalen Bilds des Kopfs des Patienten unter Verwendung eines dreidimensionalen computertomographischen Bilds,
b) Verwenden eines Segmentierungsalgorithmus zur Segmentierung des dreidimensionalen Bilds, um ein Binärbild des Gehirns zu erstellen,
c) Verwenden des Binärbilds und eines bestehenden Finite-Elemente-Modells, das in ein Binärbild umgewandelt ist, zur Erstellung einer Verformungskarte für das Finite-Elemente-Modell, Verwenden der Verformungskarte zur Versetzung der Knoten in dem bestehenden Finite-Elemente-Modell zur Anpassung an die Anthropometrie des Patienten.

7. Verfahren nach Anspruch 1, wobei die Gewebe im Kopf des Patienten unter Verwendung von Bildbearbeitungsalgorithmen klassifiziert werden.

8. Verfahren nach Anspruch 1, wobei das Erhalten des dreidimensionalen Modells das Rekonstruieren der Fließeigenschaften der ventrikulären Zerebrospinalflüssigkeit des Patienten unter Verwendung der Simulation einer Euler-Formulierung der Ventrikel des Patienten und des Verbindungskanals der Ventrikel umfasst.

9. Verfahren nach Anspruch 1, wobei das Erhalten des dreidimensionalen Modells das Erzeugen eines Finite-Elemente-Modells umfasst, bei dem der Kompressionsmodul der Zerebrospinalflüssigkeit verändert wird, um die Dehnbarkeit des zentralen Nervensystems nachzuahmen.

10. Verfahren nach Anspruch 1, ferner umfassend die Messung von Dehnungen und Spannungen im Gehirn und die Verwendung der gemessenen Dehnungen und Spannungen, um mögliche Komplikationen und Schäden am Gehirn vorherzusehen.

11. Verfahren nach Anspruch 1, ferner umfassend das Erhalten von Dehnungsmesswerten in anatomischen und histologischen Strukturen und den Vergleich der Messwerte mit der normalen Struktur zur Zuordnung zu Verletzungen des Patienten.

12. Verfahren nach Anspruch 1, wobei der Patient ein Hämatom, Ödem oder einen Tumor hat.

13. Verfahren nach Anspruch 1, wobei ein Computer mit einer Finite-Elemente-Solversoftware bei einem Verfahren zur nicht-invasiven Messung und Diagnose des intrakraniellen Zustands bei Patienten mit Anomalien aufgrund von Gehirnverletzungen verwendet wird.

14. Verfahren nach Anspruch 1, ferner umfassend das Ermitteln der Wahrscheinlichkeit weiterer Gehirnverletzungen und möglicher Ergebnisse und des möglichen Bedarfs an invasiven Maßnahmen und/oder Behandlungen.

**Revendications**

1. Procédé non-invasif d'évaluation du cerveau d'un patient, comprenant :

a) l'obtention d'images médicales du cerveau du patient ;
b) la formation d'un modèle tridimensionnel spécifique au patient du crâne et du cerveau du patient en utilisant les images médicales et un modèle numérique ;
c) la simulation d'une lésion cérébrale dans le modèle tridimensionnel spécifique au patient se basant sur le volume et le degré de la lésion visibles sur les images médicales ; et
d) l'utilisation du résultat de simulation de lésion cérébrale dans le modèle tridimensionnel pour fournir des informations sur la pression intracrânienne du patient.

**2.** Procédé selon la revendication 1, dans lequel le modèle tridimensionnel est rendu spécifique au patient en générant un nouveau modèle du cerveau du patient en utilisant une image par résonance magnétique tridimensionnelle du cerveau du patient.

**3.** Procédé selon la revendication 1, dans lequel le modèle tridimensionnel est généré automatiquement.

**4.** Procédé selon la revendication 3, dans lequel le modèle tridimensionnel est généré automatiquement par :

a) l'obtention d'une image tridimensionnelle de différents tissus cérébraux du cerveau du patient en utilisant l'imagerie par résonance magnétique ;
b) la conversion de l'image tridimensionnelle en un modèle éléments finis en transformant chaque élément d'image en un élément fini avec un volume correspondant à l'espacement de l'image tridimensionnelle ;
c) le lissage de noeuds de surface sur le modèle éléments finis pour réduire l'erreur numérique et former le modèle tridimensionnel.

**5.** Procédé selon la revendication 1, dans lequel le modèle tridimensionnel est généré en changeant un modèle existant pour l'adapter à l'anthropométrie du patient.

**6.** Procédé selon la revendication 5, dans lequel le modèle tridimensionnel est généré par

a) l'obtention d'une image tridimensionnelle de la tête du patient en utilisant une image de tomodensitométrie tridimensionnelle ;
b) l'utilisation d'un algorithme de segmentation pour segmenter l'image tridimensionnelle pour former une image binaire du cerveau ;
c) l'utilisation de l'image binaire et d'un modèle éléments finis existant, converti en une image binaire, pour créer une carte de déformation pour le modèle éléments finis ; l'utilisation de la carte de déformation pour disloquer les noeuds dans le modèle éléments finis existant pour l'adapter à l'anthropométrie du patient.

**7.** Procédé selon la revendication 1, dans lequel les tissus dans la tête du patient sont classifiés en utilisant des algorithmes de traitement d'image.

**8.** Procédé selon la revendication 1, dans lequel l'obtention du modèle tridimensionnel comprend la reproduction des propriétés d'écoulement du liquide céphalo-rachidien ventriculaire du patient en utilisant la simulation d'une formulation d'Euler des ventricules du patient et du canal de communication des ventricules.

**9.** Procédé selon la revendication 1, dans lequel l'obtention du modèle tridimensionnel comprend la création d'un modèle éléments finis dans lequel le module de compressibilité du liquide céphalo-rachidien est modifié pour imiter la souplesse du système nerveux central.

**10.** Procédé selon la revendication 1, comprenant en outre la mesure des déformations et des tensions dans le cerveau, et l'utilisation des déformations et tensions mesurées pour prévoir d'éventuelles complications et d'éventuels dommages au cerveau.

**11.** Procédé selon la revendication 1, comprenant en outre l'obtention de mesures de déformation dans des structures anatomiques et histologiques, et la comparaison des mesures à une structure normale pour les mettre en corrélation avec les lésions du patient.

**12.** Procédé selon la revendication 1, dans lequel le patient souffre d'un hématome, d'un oedème ou d'une tumeur.

**13.** Procédé selon la revendication 1, dans lequel un ordinateur avec un logiciel solveur éléments finis est utilisé dans un procédé de mesure et de diagnostic non-invasifs de l'état intracrânien pour des patients avec des anomalies dues à des lésions cérébrales.

**14.** Procédé selon la revendication 1, comprenant en outre la détermination de la probabilité d'autres lésions dans le cerveau et des résultats et besoins probables de mesures et/ou traitements invasifs.

Figure 1

Figure 2

**Figure 3**

Fringe Levels

3.000e-01
2.850e-01
2.700e-01
2.550e-01
2.400e-01
2.250e-01
2.100e-01
1.950e-01
1.800e-01
1.650e-01
1.500e-01
1.350e-01
1.200e-01
1.050e-01
9.000e-02
7.500e-02
6.000e-02
4.500e-02
3.000e-02
1.500e-02
0.000e+00

EP 2 323 548 B1

Figure 4

Figure 5

Figure 6

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6875176 B
- US 7072705 B

**Non-patent literature cited in the description**

- **GLICK, R. P. et al.** *Neurosurgery,* November 2006, vol. 59 (5), 1052-1061
- Tissue resonance analysis: a novel method for non-invasive monitoring of intracranial pressure. *J Neurosurgery,* 2002, vol. 96, 1132-1137
- **JOHAN BELLNER et al.** Transcranial Doppler sonography pulsatility index (PI) reflects intralranial pressure (ICP). *Surgical Neurology,* vol. 62 (1), 45-51
- **FARMANZAD et al.** *Bio-Medical Materials and engineering,* 2007, vol. 17, 119-125
- **SABERI et al.** *Computer Aided Surgery,* March 2007, vol. 12 (2), 131-136
- **M. SCHILL ; M. SCHINKMAN ; H.-J. BENDER ; R. MANNER.** *Biomechanical Simulation of the Falx cerebri using the Finite Element Method,* 1996
- **CHENG AY ; PAUN MC ; POON WS ; WONG GK.** *The correlation of midline shifts of human brain with large brain haematoma using a finite element approach*
- **KLEIVEN.** Predictors for traumatic brain injuries evaluated through accident reconstructions. *51st Stapp Car Crash Journal,* 2007
- **KLEIVEN.** Correlation of an FE-model of the Human Head with Local Brain Motion-Consequences for Injury Prediction. *46th Stapp Car Crash Journal,* 2002
- Influence of FE model variability in predicting brain motion and intracranial pressure changes in head impact simulations. *International Journal of Crashworthiness,* 2004, vol. 9 (4), 401-408
- **ANDERSON, R.W.G. ; BROWN, C.J. ; BLUMBERGS, P.C. ; SCOTT, G. ; FINNEY, J.W. ; JONES, N.R. ; MCLEAN, A.J.** Mechanics of axonal injury: An experimental and numerical study of a sheep model of head impact. *Proc. 1999 IRCOBI Conf. Sitges, Spain,* 1999, 107-120
- *Journal of Biomechanical Engineering,* vol. 16, 615-622
- **PUSO ; WEISS.** *J Biomech Eng.,* February 1998, vol. 120 (1), 62-70
- **FRANCESCHINI et al.** *J Mechanics and Physics of Solids,* 2006, vol. 54 (12), 2592-2620
- **NICOLLE et al.** *Biorheology,* 2005, vol. 42 (3), 209-23
- **DONNELLY.** A comparison of results. Biomechanical research: Experimental and computational. *Proc. Of the 26th int. Workshop.,* 1998, 47-57
- **ARBOGAST ; MARGULIES.** *Paper No. 973336, Society of Automotive Engineers,* 1997